# EUROPÄISCHE PATENTANMELDUNG

(11) **EP 3 553 774 A1**
(43) Veröffentlichungstag der Anmeldung: **16.10.2019**
(21) Anmeldenummer: 18166985.4
(22) Anmeldetag: 12.04.2018
(51) Int. Cl.: G10L 15/26, G10L 15/30, A41D 1/02, A61B 5/00, D06M 11/83, A41D 1/00

(54) **OBERKÖRPERBEKLEIDUNGSSTÜCK**

(71) Anmelder: S.Oliver Bernd Freier GmbH & Co. KG, 97228 Rottendorf (DE)
(72) Erfinder: Die Erfindernennung liegt noch nicht vor
(74) Vertreter: Thun, Clemens

(57) **Zusammenfassung**

Ein Oberkörperbekleidungsstück, insbesondere eine Jacke (100), ist mit einem integrierten Sprachassistenten ausgestattet, aufweisend: ein Assistentenmodul (5), welches dazu ausgebildet ist, Sprachinformationen eines Trägers des Bekleidungsstücks zu erfassen und zu bewerten sowie in den Sprachinformationen enthaltene Befehle auszuführen oder die Sprachinformationen ggf. über eine drahtlose Kommunikationsverbindung an ein externes System zum Analysieren der Sprachinformationen zu übermitteln.

## Beschreibung

Die vorliegende Erfindung betrifft ein Oberkörperbekleidungsstück, welches einen integrierten Sprachassistenten aufweist. Insbesondere betrifft die vorliegende Erfindung eine Jacke.

Sprachassistentensysteme wurden in den letzten Jahren kontinuierlich weiterentwickelt und kommen zwischenzeitlich in vielfältiger Weise zum Einsatz. Während frühere Varianten von Sprachassistenten speziell abgestimmt auf bestimmte Funktionen ausgerichtet waren, bspw. zur Steuerung des Navigationssystems eines Fahrzeugs, sind mittlerweile entsprechende Systeme auf dem Markt, welche eine universale Nutzung ermöglichen.

Ein zwischenzeitlich verbreitetes Sprachassistentensystem ist bspw. unter der Bezeichnung "Amazon Echo" bekannt und wurde zur Nutzung in privaten Wohnräumen oder vergleichbaren Situationen entwickelt. Es handelt sich hierbei um einen internetbasierten intelligenten Assistenten mit Lautsprecherfunktion, bei dem mittels Sprachsteuerung auf diverse Dienste zugegriffen werden kann. Hauptbestandteil des Systems ist eine Lautsprechereinheit, die gleichzeitig auch Mittel zum Erfassen von Sprachinformationen beinhaltet. Die Lautsprechereinheit ist hierbei derart ausgelegt, dass sie im Bereitschaftsmodus zunächst die von einem Nutzer ausgegebene Sprache geräteintern verarbeitet und auf ein Signal- oder Schlüsselwort hin überprüft. Mittels dem entsprechenden Schlüsselwort oder durch Drücken einer Aktionstaste wird die eigentliche Sprachsteuerung initiiert. Im Folgenden überträgt dann die Lautsprechereinheit die im Raum gesprochenen Worte digital zu einem Rechencenter des Anbieters, in dem die Informationen analysiert werden und versucht wird, die hierbei erkannten Befehle umzusetzen. Das Umsetzen eines Befehls erfolgt dann beispielsweise dadurch, dass durch den Befehl angeforderte Informationen wiederum auf digitalem Wege zurück an die Lautsprechereinheit übermittelt werden, welche diese dann bspw. in Form von Sprache oder Musik ausgibt oder ggf. mit der Lautsprechereinheit gekoppelte Geräte entsprechend ansteuert. Die Übermittlung der digitalen Daten zwischen Lautsprechereinheit und Rechenzentrum erfolgt üblicherweise über das Internet, lokal ist die Lautsprechereinheit über ein WLAN-Netzwerk oder eine anderweitige drahtlose Kommunikationsverbindung mit dem Internet verbunden.

Eine Gemeinsamkeit des soeben beschriebenen Sprachassistentensystems sowie anderer bekannter Sprachassistenten besteht darin, dass diese lediglich für einen stationären Betrieb ausgelegt sind. Der vorliegenden Erfindung liegt deshalb die Aufgabenstellung zugrunde, eine Möglichkeit anzubieten, derartige Sprachassistentensysteme vielseitiger, insbesondere auch mobil nutzen zu können.

Die Aufgabe wird durch ein Oberkörperbekleidungsstück, welches die Merkmale des Anspruchs 1 aufweist, gelöst. Vorteilhafte Weiterbildungen der Erfindung sind Gegenstand der abhängigen Ansprüche.

Erfindungsgemäß wird vorgeschlagen, die Kernbestandteile eines Sprachassistentensystems in ein Oberkörperbekleidungsstück zu integrieren, wobei es sich bei diesem Oberkörperbekleidungsstück insbesondere um eine Jacke handelt. Kernbestandteil des erfindungsgemäßen Sprachassistentensystems ist hierbei ein sog. Assistentenmodul, welches dazu ausgebildet ist, Sprachinformationen eines Trägers des Bekleidungsstücks zu erfassen und zu bewerten. Das Assistentenmodul ist ferner dazu ausgebildet, in den Sprachinformationen enthaltene Befehle auszuführen oder die Sprachinformationen digital über eine drahtlose Kommunikationsverbindung an ein externes System zum Analysieren der Sprachinformationen zu übermitteln.

Gemäß der vorliegenden Erfindung wird also ein Oberkörperbekleidungsstück, insbesondere eine Jacke, mit einem integrierten Sprachassistenten vorgeschlagen, aufweisend ein Assistentenmodul, welches dazu ausgebildet ist, Sprachinformationen eines Trägers des Bekleidungsstücks zu erfassen und zu bewerten sowie in den Sprachinformationen enthaltene Befehle auszuführen oder die Sprachinformationen über eine drahtlose Kommunikationsverbindung an ein System zum Analysieren der Sprachinformationen zu übermitteln.

Das Assistentenmodul übernimmt also die Funktion der bei dem oben beschriebenen bekannten System genutzten Lautsprechereinheit. Im Rahmen der vorliegenden Erfindung ist allerdings vorzugsweise vorgesehen, dass die Mittel zum Erfassen der Sprachinformationen sowie zum Ausgeben von Sprachinformationen oder anderweitigen akustischen Informationen nicht Bestandteil des Assistentenmoduls selbst sondern anderweitig in das Bekleidungsstück integriert sind. Insbesondere kann also vorgesehen sein, dass das Bekleidungsstück ein mit dem Assistentenmodul verbundenes Mikrofon oder einen mit dem Assistentenmodul verbunden Anschluss für ein Mikrofon zum Erfassen der Sprachinformationen aufweist, wobei dann insbesondere die Verbindung von dem Assistentenmodul zu dem Mikrofon oder zu dem Anschluss für das Mikrofon über eine in das Bekleidungsstück integrierte Leitung erfolgt.

In gleicher Weise ist auch vorzugsweise vorgesehen, dass das Bekleidungsstück mit dem Assistentenmodul verbundene Kopfhörer oder Anschlüsse für Kopfhörer aufweist, wobei wiederum die Verbindung von dem Assistentenmodul zu dem Kopfhörer oder zu dem Anschluss für den Kopfhörer über eine in das Bekleidungsstück integrierte Leitung erfolgt.

Die Ausgabe von Sprachinformationen oder anderen akustischen Informationen muss allerdings nicht zwingend über Kopfhörer erfolgen, sondern es kann alternativ oder ergänzend hierzu auch vorgesehen sein, dass in das Bekleidungsstück Lautsprecher integriert sind, wobei in diesem Fall das Assistentenmodul vorzugsweise dazu ausgebildet ist, die Lautsprecher drahtlos, bspw. über eine Bluetooth-Verbindung anzusteuern.

Wie auch bei dem oben beschriebenen bekannten Sprachassistentensystem wird das Assistentenmodul erfasste Sprachinformationen überwiegend nicht selbständig analysieren, sondern stattdessen nach Erkennen eines Schlüsselwortes die Informationen an das externe System zum Analysieren in digitaler Form übermitteln. Hierzu ist das Aufbauen einer Kommunikationsverbindung erforderlich, wobei das Assistentenmodul vorzugsweise dazu ausgebildet ist, das Übermitteln der Informationen über ein Mobilfunkgerät vorzunehmen, welches von dem Träger des Bekleidungsstücks genutzt wird. In diesem Fall dient also vorzugsweise das Mobiltelefon des Nutzers dazu, die Datenverbindung zu dem System zum Analysieren der Sprachinformationen aufzubauen, wobei eine Verbindung zwischen Assistentenmodul und Mobilfunkgerät wiederum vorzugsweise über eine Bluetooth-Verbindung erfolgt. Alternativ hierzu könnte das Assistentenmodul das Mobilfunktelefon allerdings auch als sog. WLAN-Hotspot nutzen oder unmittelbar ein WLAN-Netzwerk kontaktieren.

Eine andere vorteilhafte Weiterbildung der Erfindung besteht darin, dass das Bekleidungsstück von dem Assistentenmodul getrennte Energiespeichermittel aufweist, die zur Energieversorgung des Assistentenmoduls genutzt werden können. Dabei kann dann insbesondere vorgesehen sein, dass zusätzlich mit den Energiespeichermitteln gekoppelte Energiesammelmittel vorhanden sind. Es kann sich hierbei bspw. um an einer Oberfläche des Bekleidungsstücks vorgesehene Solarmodule oder vergleichbare Elemente handeln, welche zur Verfügung stehendes Sonnenlicht bzw. allgemein Licht in Energie umsetzen und die Energiespeichermittel aufladen. Ein langfristiger Betrieb des Sprachassistentensystems wird hierdurch ermöglicht.

Insgesamt wird also mit Hilfe der vorliegenden Erfindung eine flexible Nutzung eines Sprachassistenten ermöglicht, wobei insbesondere eine ortsabhängige Nutzung des Systems gewährleistet ist.

Nachfolgend soll die Erfindung anhand der beiliegenden Zeichnung näher erläutert werden. Es zeigen:
- Figur 1: die Ansicht eines erfindungsgemäßen Oberkörperbekleidungsstücks, welches einen integrierten Sprachassistenten aufweist; und
- Figur 2: eine schematische Darstellung der verschiedenen Komponenten des Sprachassistentensystems.

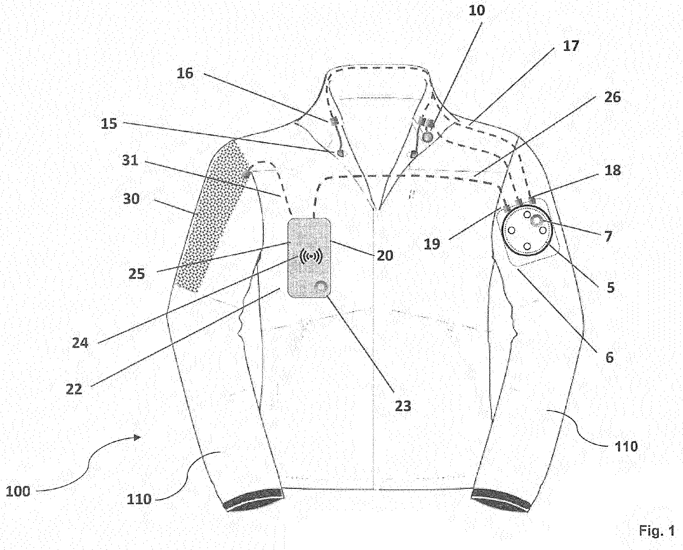

Als bevorzugtes Ausführungsbeispiel der Erfindung ist in Figur 1 eine Jacke gezeigt, die mit einem integrierten Sprachassistenten ausgestattet ist. Grundsätzlich wäre denkbar, dass Sprachassistentensystem in verschiedenste Oberkörperbekleidungsstücke zu integrieren. Es bietet sich allerdings insbesondere an, hierzu ein langärmliges Oberkörperbekleidungsstück zu verwenden, da insgesamt gesehen mehr Bereiche zur Positionierung der verschiedenen Einzelkomponenten des Systems zur Verfügung stehen. Weiterhin ist es von Vorteil, wenn das Material des Oberkörperbekleidungsstücks derart ist, dass die einzelnen Komponenten insbesondere vor Feuchtigkeit geschützt sind. Dies bedeutet allerdings nicht zwingend, dass das gesamte Bekleidungsstück aus einem entsprechenden bspw. wasserabweisenden Material bestehen muss, sondern es könnte auch vorgesehen sein, dass die einzelnen Komponenten in entsprechend geschützten Taschen positioniert werden.

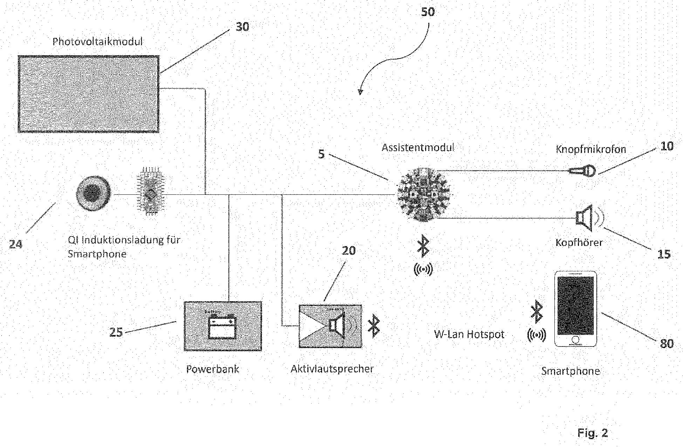

Die in Figur 1 allgemein mit dem Bezugszeichen 100 versehene Jacke weist also wie dargestellt verschiedene integrierte Komponenten auf, die insgesamt ein Sprachassistentensystem zur mobilen Nutzung bilden. Die einzelnen Komponenten des allgemein mit dem Bezugszeichen 50 versehenen Systems sind schematisch auch in Figur 2 dargestellt, wobei dieser Darstellung auch entnommen werden kann, in welcher Weise die verschiedenen Komponenten miteinander verbunden sind oder zusammenwirken.

Zentrales Element des Sprachassistentensystems 50 ist hierbei das sog. Assistentenmodul 5, welches bei dem Ausführungsbeispiel gemäß Figur 1 an einem der beiden Ärmel 110, vorzugsweise am linken Ärmel 110 der Jacke 100 angeordnet ist. Diese Anordnung hat sich als ergonomisch günstig erwiesen, da - wie nachfolgend noch näher beschrieben - zumindest optional auch eine manuelle Aktivierung des Assistentenmoduls 5 vorgesehen sein kann und eine Positionierung am linken Ärmel 110 für Rechtshänder die günstigere Anordnung darstellt. Alternativ hierzu wäre allerdings auch eine Positionierung des Assistentenmoduls 5 bspw. im zentraleren Bereich der Jacke 100 auf Brusthöhe denkbar.

Das Assistentenmodul 5 stellt wie bereits erwähnt die zentrale Einheit des Sprachassistenten 50 dar. Wie auch bei dem eingangs beschriebenen bekannten Sprachassistentensystem für den Gebrauch in Wohnräumen ist vorgesehen, dass das Assistentenmodul 5 zunächst vorab eine Auswertung von Sprachinformationen des Benutzers, also im vorliegenden Fall des Trägers der Jacke 100 vornimmt und dann entscheidet, in welcher Weise diese Sprachinformationen weiterverarbeitet werden. Üblicherweise ist hierbei vorgesehen, dass nach Erkennen eines Schlüsselworts die entsprechenden akustischen Informationen digital an ein externes, in den Figuren nicht dargestelltes System weitergeleitet werden, in dem eine detaillierte Analyse der Sprachinformationen erfolgt. Hier wird dann versucht, im Rahmen der Analyse erkannte Sprachbefehle zu beantworten, was üblicherweise in Form der Rückübermittlung von Daten erfolgt, die dann von dem System 50 bspw. also Sprache oder auch in Form von Musik oder anderen akustischen Informationen ausgegeben werden.

Das Assistentenmodul 5 kann hierbei derart ausgelegt sein, dass es dauerhaft akustische Informationen empfängt und auf das Vorhandensein von Schlüsselworten hin überprüft. Werden diese Schlüsselworte erkannt, wird dies als Sprachbefehl durch den Benutzer interpretiert und dann wie oben beschrieben eine Übermittlung der akustischen Informationen in digitaler Weise an das externe System vorgenommen. Alternativ oder ergänzend hierzu kann allerdings auch vorgesehen sein, dass wie oben bereits erwähnt der Benutzer manuell das Sprachassistentensystem 50 aktiviert, wobei dann das Assistentenmodul 5 die über das Mikrofon erfassten Daten auch ohne das Erkennen von Schlüsselwörtern unmittelbar an das zentrale System weiterleitet. Bei dieser Variante ist nicht zwingend erforderlich, dass das Assistentenmodul 5 dauerhaft akustische Signale auf das Vorliegen von Schlüsselwörtern hin überwacht, sodass insgesamt der Stromverbrauch reduziert werden kann. Weiterhin muss auch nicht zwingend vorgesehen sein, dass in den Sprachinformationen ggf. enthaltene Befehle ausschließlich durch das externe System analysiert und ausgeführt bzw. beantwortet werden. Einfache Befehle wie bspw. das Ausschalten des Systems, das Aktivieren oder Deaktivieren der Lautsprecher (bzw. ein Wechsel zwischen den zur Verfügung stehenden Lautsprechern) oder das Anpassen der Lautstärke könnte ggf. auch unmittelbar durch das Assistentenmodul 5 durchgeführt werden. Generell ist es sinnvoll, wenn Befehle zum Steuern des Sprachassistentensystems 50 durch das System 50 selbst erkannt und unmittelbar durch dieses ausgeführt werden.

Aus der obigen Schilderung der grundsätzlichen Funktionsweise des Sprachassistentensystems 50 ergibt sich die Notwendigkeit bestimmter Zusatzkomponenten, die mit dem Assistentenmodul 5 zusammenarbeiten müssen.

Hierbei ist zunächst ein Mikrofon 10 bzw. ein vergleichbares Mittel zu nennen, mit dessen Hilfe von dem Benutzer ausgegebene Sprache erfasst und an das Assistentenmodul 5 weitergeleitet werden kann. Die Positionierung des Mikrofons 10 sollte hierbei derart erfolgen, dass dieses möglichst in guter Qualität und störungsfrei von dem Träger der Jacke 100 ausgegebene Sprache erfassen kann. Vorzugsweise ist also das Mikrofon 10 in Form eines Knopfmikrofons ausgebildet, welches in der Nähe des Kopfs des Jackenträges, also beispielsweise am Kragen der Jacke 100 positioniert ist. Ggf. könnte allerdings auch das Assistentenmodul 5 unmittelbar mit einem entsprechenden Mikrofon bestückt sein.

Weiterhin ist zur Ausgabe von Sprachinformationen oder anderweitiger akustischer Informationen in Rückmeldung auf Sprachbefehle das Vorhandensein von Lautsprechern erforderlich, wobei im dargestellten Ausführungsbeispiel zwei Möglichkeiten hierfür gezeigt sind.

Zum einen können Kopfhörer 15 vorgesehen sein, die mit dem Assistentenmodul 5 verbunden und dauerhaft integriert in die Jacke 100 oder abnehmbar von dieser ausgestaltet sind. Im bevorzugten Ausführungsbeispiel abnehmbarer Kopfhörer 15 sind entsprechende Anschlüsse 16 vorgesehen, die über eine Verbindungsleitung 17 mit dem Assistentenmodul 5 verbunden sind. Diese Verbindungsleitung 17 kann insbesondere in Form eines leitfähigen Garns realisiert werden, der in das Textilmaterial der Jacke 100 eingestrickt wurde. Die Anschlüsse 16 sind dann jeweils vorzugsweise in Form eines sog. Powerklettkontakts ausgestaltet, der das einfache Anschließen oder auch Lösen der Kopfhörer 15 ermöglicht, wobei sowohl eine mechanische Befestigung als auch eine elektrische Kontaktierung realisiert wird.

Diesbezüglich ist anzumerken, dass auch das Assistentenmodul 5 nicht dauerhaft in die Jacke 100 integriert sein muss, sondern auch abnehmbar ausgestaltet sein kann. In diesem Fall ist das Assistentenmodul 5 in einer in Figur 1 schematisch angedeuteten abnehmbaren Tasche 6 aufgenommen, die wiederum vorzugsweise mittels eines Klettverschlusses lösbar an dem Ärmel 110 befestigt sein kann. Auch hier sind dann entsprechende Powerklettkontakte 18 bzw. 19 vorgesehen, über die eine elektrische Verbindung zu in die Jacke 110 integrierten Leitungen 17 erfolgt.

Alternativ oder ergänzend zu den erwähnten Kopfhörern 15 könnte allerdings eine Ausgabe der Sprachinformationen oder akustischen Daten durch das Sprachassistentensystem 50 auch über einen Aktivlautsprecher 20 erfolgen, der im dargestellten Ausführungsbeispiel im Brustbereich der Jacke 100 angeordnet ist. Das Assistentenmodul 5 übermittelt in diesem Fall dann die entsprechenden Daten an den Aktivlautsprecher 20, wobei vorzugsweise vorgesehen ist, dass die Datenübermittlung drahtlos erfolgt. Hierzu kann insbesondere die Nutzung einer Bluetooth-Verbindung vorgesehen sein, wobei das Assistentenmodul 5 und der Aktivlautsprecher 20 dann für eine entsprechende Bluetooth-Kommunikation ausgelegt sind. Die Auswahl, ob die Kopfhörer 15 oder Aktivlautsprecher 20 für die Ausgabe von Sprachinformationen oder akustischen Daten genutzt werden, kann bspw. mit Hilfe eines Sprachbefehls oder auch einer entsprechenden Betätigung von Funktionstasten an dem Assistentenmodul 5 getroffen werden. Ferner kann alternativ zu einer drahtlosen Verbindung auch vorgesehen sein, dass die Datenübermittlung über eine weitere in die Jacke 100 integrierte Leitung kabelgebunden erfolgt. Diese optionale weitere Leitung ist im dargestellten Ausführungsbeispiel nicht gezeigt.

Die in Figur 1 erkennbare Leitung 26 dient nämlich der Stromversorgung des Assistentenmoduls 5, da die den Aktivlautsprecher 20 beinhaltende Einheit 22 gleichzeitig auch eine sog. Powerbank 25, also eine Energiespeichereinheit beinhaltet. Diese ist insbesondere in Form eines Akkumulators ausgeführt und stellt die zentrale Energieversorgungsquelle des Systems 50 dar. Dabei ist ferner vorgesehen, dass die Energiespeichereinheit 25 auch zum Aufladen eines Mobilfunktelefons bzw. Smartphones genutzt werden kann. Hierzu ist die Einheit 22 zusätzlich mit einem Induktionsladungsmodul (QI) 24 versehen, über das ein Aufladen eines entsprechenden Mobilfunktelefons ermöglicht wird. Eine nicht näher gezeigte Jackentasche für das Mobilfunktelefon ist dann idealerweise derart positioniert dass sich das QI-Modul 24 der Energiespeichereinheit 25 mittig direkt unter dem Mobilfunktelefon befindet.

Auch in diesem Fall kann die kombinierte Energiespeicher-/ Lautsprechereinheit 22 von der Jacke 100 abnehmbar gestaltet sein, wobei dies ist allerdings nicht zwingend erforderlich ist. Weiterhin ist selbstverständlich auch nicht zwingend erforderlich, dass die Energiespeichereinheit 25 und der Aktivlautsprecher 20 in dem dargestellten gemeinsamen Kombigerät 22 zusammengefasst sind. Auch eine Trennung beider Einheiten wäre denkbar, wobei dann bspw. ein oder mehrere Lautsprecher auch im Schulterbereich der Jacke 100 positioniert sein könnten.

Die Energiespeicher-/ Lautsprechereinheit 22 ermöglicht dabei ferner auch das Erstellen einer Audioverbindung zum einem - lediglich in Figur 2 gezeigten - Mobilfunktelefon des Benutzers. Hierzu weist das Kombigerät 22 eine Nahfeldkommnikationseinheit, also einen NFC-Chip 23 auf, mit dessen Hilfe bei Annäherung eines Mobilfunktelefons bzw. Smartphones eine Audioverbindung - beispielsweise über Bluetooth zwischen dem Smartphone und dem Lautsprecher hergestellt wird, um z.B. Musik mit dem Smartphone über den Lautsprecher 20 wiederzugeben.

Eine vorteilhafte Weiterbildung der Erfindung kann ferner auch darin bestehen, dass die Jacke 100 selbst die Möglichkeit bietet, die Energiespeichereinheit 25 während des Tragens der Jacke 100 aufzuladen. Im vorliegenden Fall ist hierbei schematisch dargestellt, dass einer der beiden Ärmel 110 im Oberarmbereich an seiner Außenseite mit einem Photovoltaikmodul 30 auf Textilbasis bzw. in Form eines Dünnfilms ausgestaltet ist. Wichtig ist, dass das Photovoltaikmodul 30 eine gewisse Flexibilität aufweist, sodass der Tragekomfort der Jacke 100 nicht negativ beeinträchtigt wird. Auch hier ist allerdings die Anordnung des Photovoltaikmoduls 30 am Oberarmbereich eines Ärmels 110 lediglich beispielhaft zu verstehen und es wäre auch eine Anordnung im Rücken- oder Schulterbereich der Jacke 100 denkbar. Das Photovoltaikmodul 30 ist hierbei über eine weitere Leitung 31 mit der Energiespeichereinheit 25 verbunden, wobei wiederum die Leitung 31 in Form eines eingestrickten leitfähigen Garns realisiert sein kann und die Anschlüsse ggf. als Powerklettkontakte ausgeführt sind.

Das bislang beschriebene System 50 weist also das Assistentenmodul 5, Mittel zum Erfassen von Sprachinformationen in Form des Mikrofons 10 sowie zur Ausgabe von Sprach- oder anderweitigen akustischen Informationen in Form der Kopfhörer 15 bzw. des Lautsprechers 20 sowie Mittel zur Energieversorgung auf. Darüberhinausgehend muss dann wie bereits erwähnt gewährleistet sein, dass das Assistentenmodul 5 in der Lage ist, komplexere Sprachinformationen in digitaler Weise an das externe System zur Bewertung und Beantwortung zu übermitteln. Hierfür kann entsprechend der Darstellung gemäß Figur 2 vorgesehen sein, dass ein von dem Träger der Jacke 100 genutztes Mobilfunktelefon 80 genutzt wird, welches die Anbindung an das Internet ermöglicht. Dieses Mobilfunktelefon 80 kann von dem Träger der Jacke 100 entweder in einer Jackentasche oder anderweitig transportiert werden, wobei eine drahtlose Kopplung zwischen Assistentenmodul 5 und Mobilfunktelefon 80 vorgesehen ist. Hierzu weist das Assistentenmodul 5 den in Figur 1 erkennbaren NFC-Chip 7 auf, der bei einer Annäherung des Mobilfunktelefons 80 einen WLAN Hotspot für eine Internetverbindung aktiviert. Das Assistentenmodul 5 verbindet sich dann automatisch mit dem Standard WLAN-Hotspot. Alternativ hierzu kann ferner auch vorgesehen sein, dass das Assistentenmodul 5 in der Lage ist, unabhängig von einem Mobilfunktelefon 80 ein WLAN-Netzwerk zu kontaktieren, sofern sich die Jacke 100 im Bereich eines entsprechenden WLAN-Netzwerks befindet.

Betriebszustände des Systems 50 können ggf. durch - in den Figuren nicht dargestellte - entsprechende LEDs oder eine anderweitige Anzeige an dem Assistentenmodul 5 darstellt werden. An der Oberseite des Moduls 5 können ferner vorzugsweise zusätzlich noch Bedienelemente in Form von Drucktasten vorgesehen sein, die ein manuelles Aktivieren oder Ansteuern des Assistentenmoduls 5 ermöglichen. Wie zuvor geschildert kann bspw. mit Hilfe der Drucktasten ein Weiterleiten der Sprachinformationen an das zentrale System forciert werden, unabhängig von der Nutzung spezieller Schlüsselwörter. Auch ein generelles Ein- oder Ausschalten kann über derartige Drucktasten erfolgen, wobei entsprechende Symbole für die Bedienung vorzugsweise auf die Oberseite der Tasche 6 gestickt oder je nach Material auch gelasert sein können.

Letztendlich ermöglicht also das Zusammenwirken der verschiedenen Komponenten des Systems 50, dass ein Sprachassistent geschaffen wird, der in herkömmlicher Weise genutzt werden kann, allerdings nicht auf bestimmte räumliche Bereiche eingeschränkt ist, sondern an nahezu beliebiger Stelle und damit deutlich flexibler verwendet werden kann.

## Patentansprüche

1. Oberkörperbekleidungsstück, insbesondere Jacke (100), mit einem integrierten Sprachassistenten, aufweisend:
ein Assistentenmodul (5), welches dazu ausgebildet ist, Sprachinformationen eines Trägers des Bekleidungsstücks zu erfassen und zu bewerten sowie in den Sprachinformationen enthaltene Befehle auszuführen oder die Sprachinformationen ggf. über eine drahtlose Kommunikationsverbindung an ein externes System zum Analysieren der Sprachinformationen zu übermitteln.

2. Oberkörperbekleidungsstück nach Anspruch 1,
**dadurch gekennzeichnet,**
**dass** dieses ein mit dem Assistentenmodul (5) verbundenes Mikrofon (10) oder einen mit dem Assistentenmodul (5) verbundenen Anschluss für ein Mikrofon (10) zu Erfassen der Sprachinformationen aufweist.

3. Oberkörperbekleidungsstück nach Anspruch 2,
**dadurch gekennzeichnet,**
**dass** die Verbindung von dem Assistentenmodul (5) zu dem Mikrofon (10) oder zu dem Anschluss für das Mikrofon (10) über eine in das Bekleidungsstück integrierte Leitung erfolgt.

4. Oberkörperbekleidungsstück nach einem der Ansprüche 1 bis 3,
**dadurch gekennzeichnet,**
**dass** dieses mit dem Assistentenmodul (5) verbundene Kopfhörer (15) oder einen Anschluss (16) für Kopfhörer (15) aufweist.

5. Oberkörperbekleidungsstück nach Anspruch 4,
**dadurch gekennzeichnet,**
**dass** die Verbindung von dem Assistentenmodul (5) zu dem Kopfhörer (15) oder zu dem Anschluss (16) für den Kopfhörer (15) über eine in das Bekleidungsstück integrierte Leitung (17) erfolgt.

6. Oberkörperbekleidungsstück nach einem der vorherigen Ansprüche,
**dadurch gekennzeichnet,**
**dass** dieses einen integrierten Lautsprecher (20) aufweist, wobei das Assistentenmodul (5) dazu ausgebildet ist, den Lautsprecher (20) vorzugsweise drahtlos, insbesondere über eine Bluetooth-Verbindung anzusteuern.

7. Oberkörperbekleidungsstück nach einem der vorherigen Ansprüche,
**dadurch gekennzeichnet,**
**dass** dieses Energiespeichermittel (25) zur Energieversorgung des Assistentenmoduls (5) aufweist.

8. Oberkörperbekleidungsstück nach Anspruch 7,
**dadurch gekennzeichnet,**
**dass** die Energiespeichermittel (25) dazu ausgebildet sind, ein Mobilfunktelefon (80) drahtlos, insbesondere über Induktion aufzuladen.

9. Oberkörperbekleidungsstück nach Anspruch 7 oder 8,
**dadurch gekennzeichnet,**
**dass** dieses ferner mit den Energiespeichermitteln (25) gekoppelte Energiesammelmittel (26) aufweist.

10. Oberkörperbekleidungsstück nach Anspruch 6 und einem der Ansprüche 7 bis 9,
**dadurch gekennzeichnet,**
**dass** der Lautsprecher (20) und die Energiespeichermittel (25) in einer Einheit zusammengefasst sind.

11. Oberkörperbekleidungsstück nach einem der vorherigen Ansprüche,
**dadurch gekennzeichnet,**
**dass** das Assistentenmodul (5) dazu ausgebildet ist, das Übermitteln der Sprachinformationen an das externe System über ein Mobilfunktelefon (80) vorzunehmen, wobei das Assistentenmodul (5) vorzugsweise über eine Bluetooth-Verbindung mit dem Mobilfunktelefon (80) koppelbar ist oder dazu ausgebildet ist, das Mobilfunktelefon (80) als WLAN-Hotspot zu nutzen.

12. Oberkörperbekleidungsstück nach einem der vorherigen Ansprüche,
**dadurch gekennzeichnet,**
**dass** in das Bekleidungsstück integrierte Leitungen (17, 26, 31) in Form eingestrickter leiterfähiger Garne realisiert sind.

13. Oberkörperbekleidungsstück nach einem der vorherigen Ansprüche,
**dadurch gekennzeichnet,**
**dass** das Assistentenmodul (5) Mittel zur Anzeige eines Betriebszustands aufweist.

14. Oberkörperbekleidungsstück nach einem der vorherigen Ansprüche,
**dadurch gekennzeichnet,**
**dass** das Assistentenmodul (5) Bedienelemente beispielsweise zum manuellen Aktivieren des Sprachassistenten aufweist.
